(19)

Europäisches
Patentamt

European
Patent Office

Office européen
des brevets

(11) **EP 2 899 659 A1**

(12) **EUROPEAN PATENT APPLICATION**

(43) Date of publication:
    **29.07.2015 Bulletin 2015/31**

(51) Int Cl.:
    **G06F 19/00** (2011.01)     **A61N 5/10** (2006.01)

(21) Application number: **15151834.7**

(22) Date of filing: **20.01.2015**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**

(30) Priority: **23.01.2014 US 201414162105**

(71) Applicant: **Mitsubishi Electric Corporation
    Chiyoda-ku
    Tokyo 100-8310 (JP)**

(72) Inventors:
    • **Brand, Matthew
      Cambridge, MA Massachusetts 02139 (US)**
    • **Ramakrishnan, Jagdish
      Cambridge, MA Massachusetts 02139 (US)**

(74) Representative: **Pfenning, Meinig & Partner GbR
    Patent- und Rechtsanwälte
    Theresienhöhe 11a
    80339 München (DE)**

(54) **Method for optimizing dose of radiation for radio-therapy treatment and radiation therapy system**

(57)    A method optimizes a dose of radiation for a radio-therapy treatment subject to constraints on diagnostic parameters of the radio-therapy treatment. The method determines a point of a polytope arranged in a coordinate system of the diagnostic parameters, such that a position of the point in the coordinate system is determined at least in part by values of each diagnostic parameter. The polytope is convex with boundaries formed by intersecting half-spaces of feasible values of each diagnostic parameter specified by the constraints. The point is the closest point of the polytope to an origin of the coordinate system with regard to a weighted Euclidean distance norm. The method determines a distribution of the dose of radiation for the radio-therapy treatment using the values of the diagnostic parameters corresponding to the position of the point.

FIG. 4

**Description**

**Field of the Invention**

This invention relates to particle beam dose optimization, and more particularly to optimization of the dose subject to constraints on diagnostic parameters.

**Background of the Invention**

Radiation therapy is used to treat malignant tissue, such as cancer cells. The radiation can have an electromagnetic form, such as high-energy photons, or a particulate form, such as an electron, proton, neutron, or alpha particles. Fast and accurate dose determination is important for radiation therapy treatment planning to ensure that the correct dose is delivered to a patient. Dose determination generally includes two parts: a source model and a transport model. The source model provides the incident fluence, which is the flux of the radiation integrated over time. The transport model determines the dose that results from the incident fluence.

One object of particle beam dose optimization (PBDO) is to prevent an under-dose to the tumor and over-dose to organs-at-risk (OARs) and healthy tissue during a radio-therapy treatment. The treatment constraints for irradiation are usually prescribed by a clinician responsible for the radio-therapy treatment. Often it is physically impossible to satisfy all the constraints in a prescription, in which case the optimization problem is infeasible - no solution exists. Then it becomes necessary to explore variants of the prescription to find a feasible combination of the treatment constraints without making too many compromises. Clinicians are often engaged in an iterative process of adjusting objectives and/or constraints of the PBDO until a desirable and feasible treatment plan is obtained. Multi-criterion optimization (MCO) is a framework for this process in which some hard constraints are changed to soft objectives also known as cost functions; minimizing different weighted sums of these objectives yield different optimal solutions. The set of all such optimal solutions forms a convex surface in a space where the coordinates of any candidate solution are its costs with regard to the unweighted objectives. The convex surface, known as a Pareto set or Pareto surface, is the boundary between the space of infeasible solutions and the region of suboptimal solutions.

Most conventional methods that use the Pareto surface in the PBDO follow the same outline. A large number of solutions are predetermined, e.g., in the overnight calculations. Each solution optimizes a different feasible weighting of the objectives, and the total number of such solutions forms a point-wise sampling of the Pareto surface. Interpolating between nearby points gives suboptimal solutions that are near the Pareto surface. A real-time interface is provided for the clinician to examine the interpolated surface to select a desired solution, see, e.g., U.S. 8,315,357 or U.S. 2009/037,150.

However, clinicians find representations of the Pareto surface difficult to understand and to analyze. Instead of trying to visualize the Pareto surface, recent systems represent individual criteria or trade-offs with familiar graphical user interface elements such as dials or sliders. However, those criteria have no physical meaning in the radio-therapy treatment, and can be counter intuitive or even confusing. In addition, the selection of the criteria results in an approximate solution that deviates from the Pareto surface. After the approximate solution has been selected, re-optimization methods are repeated to determine a nearby point on the real Pareto surface. The result may not preserve the properties that were the basis of the clinician's final selection.

The need for predetermining the Pareto surface and selecting an approximation of the feasible solution with subsequent re-optimization makes the PBDO computationally inefficient, and inaccurate.

**Summary of the Invention**

Various embodiments of the invention are based on a realization that dose optimization for radiation therapy can be cast as a weighted Least-Distance Problem (LDP) between an origin of a coordinate system parameterized on the space of possible solutions of the radio-therapy treatment problem and a convex feasible polytope arranged in that coordinate system with boundaries formed by intersecting half-spaces of feasible values of each diagnostic parameter specified by the constraints. In one embodiment, the weighting is selected such that the distance represents the total irradiation to the patient, either exactly or in approximation.

The point on the polytope that is closest to the origin belongs to a Pareto surface of possible solutions satisfying the constraints on diagnostic parameters. Notably, because the problem cast as the LDP, there is no need to explicitly determine the polytope or the Pareto surface.

The shape of the polytope of the LDP is determined by the diagnostic parameters, such that each facet of the polytope represents a set of solutions where one clinical constraint is satisfied exactly, and the interior of the polytope represents a set of solutions where all clinical constraints are satisfied with some room for error. For example, the diagnostic parameters and the constraints on the diagnostic parameters can include a constraint on a minimal irradiation of a tumor, a constraint on a maximal irradiation of at least one organ-at-risk (OAR), and a constraint on a maximal irradiation of normal tissues.

Some embodiments use various optimization methods to solve the LDP without determining all possible solutions forming the polytope. For example, because the polytope is arranged in the coordinate system of radiation source intensities and the total irradiation is related to the intensity values of beams of radiation, the LDP problem can be formulated as minimizing a weighted norm of the intensity values of the beams of radiation. Also, various constraints on the diagnostic parameters can be formulated as minimal and maximal constraints on the total irradiation of various voxels of a treatment volume. Such formulation allows deriving a mathematical representation of the LDP susceptible to various optimization methods that do not require construction of the entire Pareto surface.

After a position of the closest point of the polytope to the origin is found, the values of the diagnostic parameters specifying the position of the closest point can be used to determine a distribution of a dose of radiation. Because of the parameterization on the diagnostic parameters, it is guaranteed that the found values of the diagnostic parameters correspond to the feasible solution that minimizes a weighted norm of the total irradiation. In addition, the parameterization on the diagnostic parameters provides an opportunity for the clinician to directly explore effects of changing constraints on the diagnostic parameters on the distribution of a dose of radiation.

Notably, the change in at least one constraint on at least one diagnostic parameter can change the shape of the polytope and thus change the position of the polytope point closest to the origin. But the updated position is still on the polytope and still corresponds to an optimal solution. Moreover, in this framework it is possible to determine whether the new optimal point lies on the same facet as a previously optimal point, and in such cases determine the new optimal solution without performing optimization. Thus, the clinician by varying the constraints on the diagnostic parameters that have physical and medical meaning can directly determine optimal and feasible combination of the diagnostic parameters without the need to resort to approximations and/or the need to reconstruct and/or approximate the Pareto surface.

Moreover, various embodiments of the invention transform the LDP into a dual problem in order to use a parallel quadratic programming (PQP) method, which iteratively rescales a candidate solution of the optimization problem, and which takes full advantage of parallel multi-processors computation. Such reformulation of the LDP allows determining the optimal and feasible solution in real time, which provides clinicians with unique opportunity to explore effects of different constraints on diagnostic parameters in real time.

Accordingly, one embodiment discloses a method for optimizing a dose of radiation for a radio-therapy treatment subject to constraints on diagnostic parameters of the radio-therapy treatment. The method includes determining a point of a polytope arranged in a coordinate system of the diagnostic parameters, such that a position of the point in the coordinate system is determined at least in part by values of each diagnostic parameter, wherein the polytope is convex with boundaries formed by intersecting half-spaces of feasible values of each diagnostic parameter specified by the constraints, and wherein the point is the closest point of the polytope to an origin of the coordinate system with regard to a weighted Euclidean distance norm; and determining a distribution of the dose of radiation for the radio-therapy treatment using the values of the diagnostic parameters corresponding to the position of the point. The steps of the method are performed by a processor.

Another embodiment discloses a method for optimizing a dose of radiation for a radio-therapy treatment of a treatment volume of a patient subject to constraints on diagnostic parameters of the treatment volume. The method includes determining minimal and maximal constraints on total dose of radiation of each voxel in the treatment volume based on the constraints and a voxel map of the treatment volume; formulating a least-distance problem (LDP) minimizing intensity values of beams of radiation subject to the minimal and the maximal constraints on the total dose of radiation of each voxel in the treatment volume; taking a dual of the LDP to transform the LDP into a non-negative quadratic program (NNQP); solving the NNQP using a parallel quadratic programming (PQP) rescaling iteratively a candidate solution of the NNQP to determine a dual solution; and determining a primal solution of the LDP using the dual solution of the NNQP. The steps of the method are performed by a processor.

Yet another embodiment discloses a radiation therapy system, including a processor for optimizing a dose of radiation for a radio-therapy treatment of a treatment volume of a patient subject to constraints on diagnostic parameters of the treatment volume, wherein the processor is configured for determining a solution of a least-distance problem (LDP) minimizing intensity values of beams of radiation subject to minimal and maximal constraints on the dose of radiation of each voxel in the treatment volume to produce optimal values of the diagnostic parameters satisfying the constraints, and for determining a distribution of the dose of radiation for the radio-therapy treatment using the optimal values of the diagnostic parameters.

**Brief description of the Drawing**

Figure 1 is a schematic diagram of a radiation therapy system according to one embodiment of the invention;

Figure 2 is an exemplar polytope arranged in the coordinate system according to one embodiment of the invention;

Figure 3 is a schematic of a two-dimensional (2D) representation of the polytope in Figure 2;

Figure 4 is a block diagram of a method for determining the position of the point of the polytope according to one embodiment of the invention;

Figure 5 is a block diagram of a method for updating the position of the point according to some embodiments of the invention;

Figure 6A is an example of a Pareto curve according to one embodiment of the invention;

Figure 6B is an example of such a Pareto curve for analyzing a slack variable according to one embodiment of the invention;

Figure 7 is a graph comparing a difference between PQP update iteration, and various acceleration techniques according to some embodiments of the invention; and

Figures 8A and 8B are examples of rendering the distribution of the dose of radiation according to one embodiment of the invention;

**Detailed Description of the Preferred Embodiments**

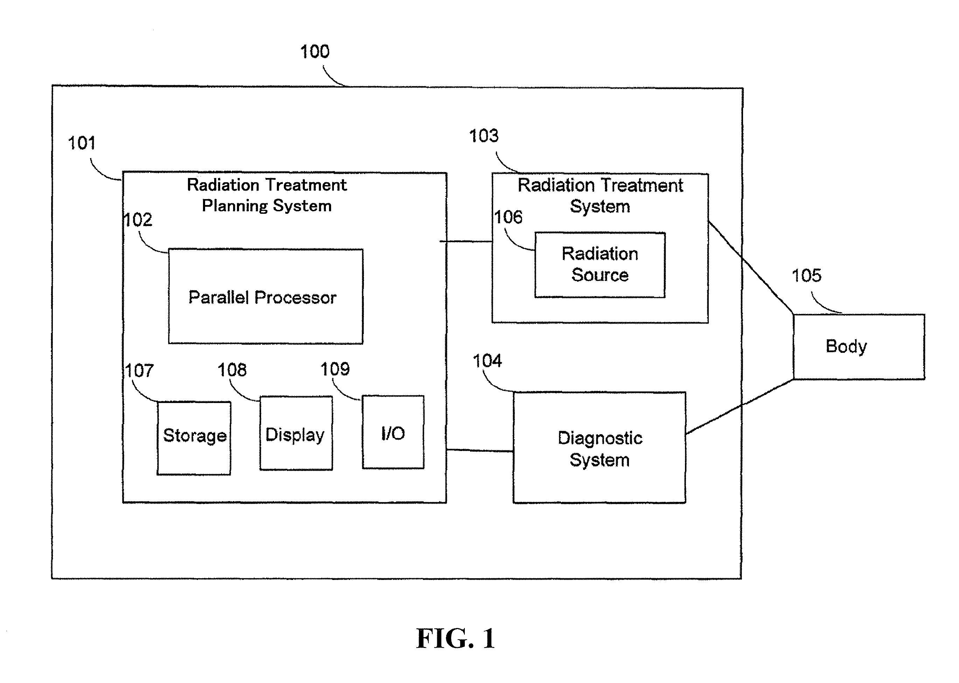

Figure 1 is a schematic diagram of a radiation therapy system 100 according to one embodiment of the invention. The radiation therapy system 100 includes a radiation treatment planning system 101, which further includes a parallel processor 102. The parallel processor is adapted to receive input information concerning a body 105 having an intended radiation treatment volume that can be represented as a volume of voxels. The parallel processor 102 is also adapted to generate output information for providing radiation treatment to the intended radiation treatment volume of the body.

The radiation treatment planning system 101 can further include a storage 107, a display 108, and input/output (I/O) devices and interfaces 109. The storage 107 may be, for example, a hard disk drive, a CD-ROM drive, a DVD drive, a flash drive, etc. The display 108 may be, for example, a liquid crystal display (LCD), a cathode ray tube (CRT) monitor, a plasma display, etc. I/O device 109 may include, for example, a mouse, a keyboard, an interface for data transfer over a network or a data bus.

The radiation therapy system 100 can further include a radiation treatment system 103 that is in communication with the radiation treatment planning system 101. The radiation treatment system 103 can include a radiation source 106 that emits a plurality of directed beams of radiation for treatment. Examples of radiation sources may include an X-ray source, a gamma ray source, an electron beam source, etc. The radiation source 106 may further comprise a multi-leaf collimator (MLC) to shape the beam. By adjusting the position of the leaves of the MLC, a radiotherapist can match the radiation field to a shape of the treatment volume of body. Other beam shaping and/or contouring can be included in some embodiments. The radiation source 106 can have a corresponding source model. The radiation treatment system 103 may be controlled by the radiation treatment planning system 101, for example, to deliver intensity modulated radiation energy and to conform radiation treatment to the shape of the intended radiation treatment volume.

The radiation therapy system 100 can also include a diagnostic system 104, in communication with the radiation treatment planning system 101 that generates empirical data of body 105, e.g., a body of a patient. The empirical data may be used as input information to the radiation treatment planning system 101 and the parallel processor 102 and may be used to determine the dose of radiation. The diagnostic system 104 can include sensors to obtain the empirical data of body 105. An example diagnostic system may be a computed tomography (CT) scanner, a magnetic resonance imaging (MRI) scanner, a positron emission tomography (PET) scanner.

One object of radiation therapy dose optimization is to prevent under-dose to the tumor and over-dose to organs-at-risk (OARs) and other healthy tissue during a radio-therapy treatment. The radiation therapy is usually performed according a prescription of diagnostic parameters. The prescription, e.g., can provide minimum dose of radiation to each volume unit in the tumor, maximum dose to each volume unit in each nearby healthy organ. In the settings of pencil-beam radiation therapy or particle-beam radiation therapy (PBDO), one seeks to compute intensity values for a large number of radiation beams such that the diagnostic parameters in the prescription are satisfied.

Various embodiments of the invention are based on a realization that dose optimization for radiation therapy can be cast as a least-distance problem (LDP) between an origin of a coordinate system of the total irradiation parameterized on the diagnostic parameters of the radio-therapy treatment and a convex feasible polytope arranged in that coordinate system with boundaries formed by intersecting half-planes of feasible values of each diagnostic parameter specified by the constraints.

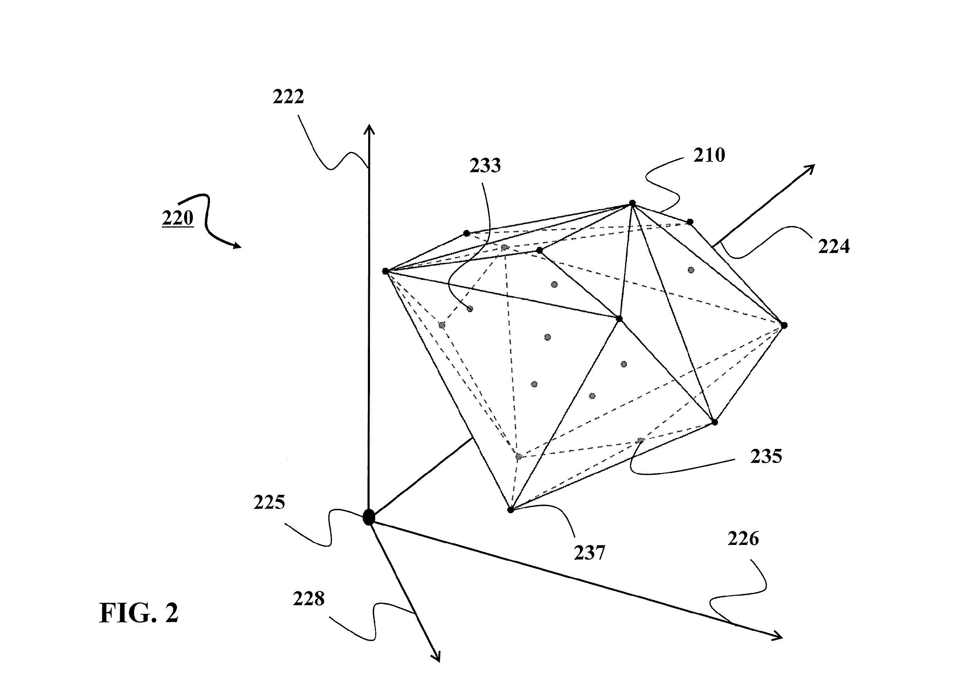

Figure 2 shows an exemplar polytope 210 arranged in the coordinate system 220 according the principles of the above realization. The coordinate system 220 is usually a low-dimensional system of diagnostic parameters. For example, if the prescription includes four constraints for four diagnostic parameters, such as minimal dose of radiation for a tumor T, maximal dose of radiation for a first and a second organs-at-risks (OAR) OAR1 and OAR2, and maximal dose of radiation for normal tissues N, the coordinate system 220 is four dimensional and have one dimension T 222 for values of radiation of the tumor subject, a dimension OAR1 224 for values of radiation for the first OAR, a dimension OAR2 226 for values of radiation for the second OAR, and a dimension 228 for values of radiation for the normal tissue. The coordinate system 220 can have other constraints and corresponding dimensions related to the physics and/or mechanics of the radiation treatment. For example, some constraint can specify the minimal possible radiation.

Such arrangement formulates the PBDO in parameters that have physical meaning to the clinicians. A set of points on the polytope, such as points 233, 235, 237 define a set of possible solutions satisfying the constraints on diagnostic parameters. However, only one point, e.g., the point 237 closest to the origin 225 representing zero radiation, specifies the optimal solution minimizing the total irradiation of the patient. Here "closest" is interpreted as minimizing some weighted convex norm, for example minimizing a suitably weighted Euclidean norm minimizes the total irradiation exactly whereas minimizing an unweighted Euclidean norm minimizes an upper bound on total irradiation. Accordingly, the PBDO is formulated as the LDP between the origin 225 and the polytope 210.

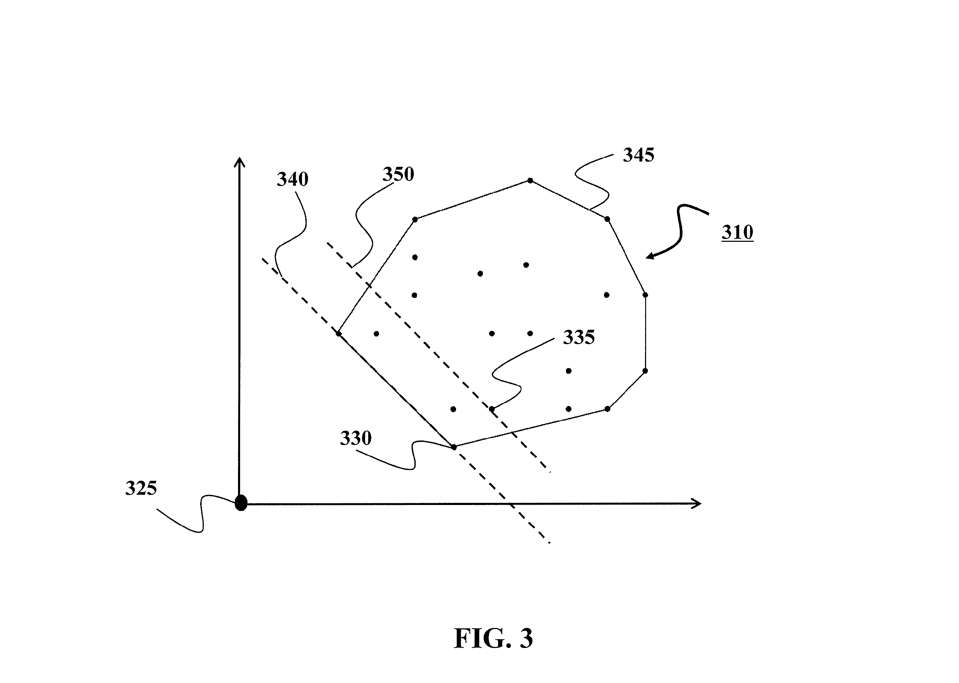

Figure 3 shows a two-dimensional (2D) representation 310 of the polytope 210 in the 2D coordinate system. Each line, e.g., lines 340 and 345, forming the border of the polytope correspond to the constraint on the diagnostic parameter, i.e., half-planes forming the polytope 210.

The change in at least one constraint on at least one diagnostic parameter can change the position of the closest point on the polytope. For example, a change of a value of a constraint represented by a line 340 to a value represented by a line 350 changes a shape of the polytope and updates a position of the closests to the original point from the position 330 to the position 335. But the updated position of the point is still on the polytope, and thus corresponds to a feasible solution, and still closest to the origin and corresponds to an optimal solution. Thus, the clinician by varying the constraints on the diagnostic parameters that have physical and medical meaning can directly determine optimal and feasible combination of the diagnostic parameters without the need to resort to approximations and/or the need to reconstruct the Pareto surface.

**Particle beam dose optimization as a least-distance problem (LDP)**

Some embodiments use various optimization methods to solve the LDP without explicitly determining the polytope that represents minimal and maximal constraints on the total irradiation of various voxels of a treatment volume. Such formulation allows deriving a mathematical representation of the LDP susceptible to various optimization methods. For example, the LDP can be transformed into a mathematically equivalent dual formulation as a non-negative quadratic program (NNQP), where the clinical constraints that determine the LDP polytope are represented by a quadratic cost function that is to be minimized over the space of non-negative vectors, and each element of one such vector represent a cost of satisfying one of the clinical constraints. The PQP method can solve this NNQP very rapidly, and the optimal solution of the LDP is a linear transform of the optimal solution of the NNQP. However, it is not practical to explicitly represent this quadratic cost function, even on present-day supercomputers. Some embodiments use a new form of PQP that specifically solves LDP problems without explicitly forming the quadratic cost function.

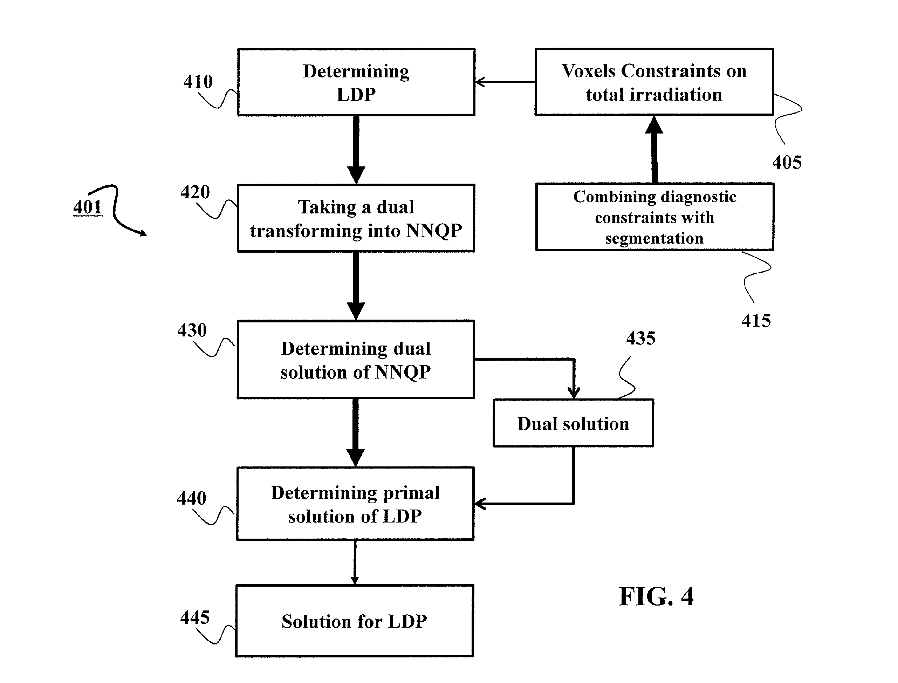

Figure 4 shows a block diagram of a method for determining the position of the point of the polytope closest to the origin by solving an optimization problem without constructing the polytope. The method can be implemented using a processor 401, e.g., a parallel processor 102.

The method combines 415 the prescription with a segmentation of a treatment volume of the patient to be irradiated, e.g., a voxel map where each voxel is labeled as "tumor," "liver," "bone," etc., and a fluence matrix, i.e., a table which determines the rate at which each beam deposits radiation into each voxel. Typically each labeled entity spans a 3D volume containing thousands of voxels. Using the segmentation, the prescription is converted 415 to constraints 405 on each and every voxel in the treatment planning volume.

The generic optimization problem is to set the intensity values of many beams such that their combined radiation pattern satisfies each constraint for each voxel minimizing the total irradiation. The problem, thus stated, is a linear program (LP). However, LPs of this size are typically too large to be solved in a practical amount of time. A further demerit of the LP approach is that the LP solution is not spatially smooth, which leads to incorrect dosing when the patient is misaligned at treatment time. In addition, the heuristic optimizations, e.g., penalized least-squares, are relatively fast and yield smooth solutions, but do not necessarily satisfy the constraints.

Some embodiments of the invention are based on a realization that the optimization problem can be formulated 410 as a weighted LDP, in which the constraints are satisfied exactly while minimizing a weighted L2 norm of the beam intensity values, which promotes spatially smoother solutions. In addition, the weighting can be selected to make the optimal LDP solution similar or identical to the optimal LP solution. Geometrically, for LP and LDP, the constraints form a convex polytope in the space of beam weight vectors; each constraint defines a half-space and the polytope is the intersection of all such half-spaces. In LDP the goal is to find a point in this polytope that is closest to the origin.

Some embodiments use the following LDP formulation:

$$\min_{x \geq 0} \frac{1}{2} \| Wx \|_2^2 \quad \text{s.t.} \quad b_{\min} \leq Ax \leq b_{\max}, \qquad (2.1)$$

where $A \in \mathbb{R}^{m \times n} \geq 0$ is a non-negative dose-fluence matrix that maps a vector $x \in \mathbb{R}^n \geq 0$ of beam intensity values to a vector $Ax \in \mathbb{R}^m$ of cumulative voxel doses, and $W \in \mathbb{R}^{n \times n} \geq 0$ is a diagonal matrix of weights. The lower bound constraint $Ax \geq b_{\min}$ sets a minimum prescription dose for each voxel in the tumor, and the upper bound constraint $Ax \leq b_{\max}$ is used to protect OAR voxels and prevent hot spots in the tumor.

Various weighting schemes are used for matrix W by different embodiments. For example, with $W = \text{diag}(A^Te)$, where e is a vector of ones, each element of the vector Wx includes the total dose delivered by one beam. In that case, minimizing the 2-norm $\|Wx\|_2$ is equivalent to minimizing the squared mean dose plus the dose variance (on a per-beam basis), because $E[X^2] = (E[X])^2 + \text{var}[X]$. This promotes both parsimony and smoothness in the solution.

Additionally or alternatively, with $W = \text{diag}(\hat{x})^{-1/2}\text{diag}(A^Te)^{1/2}$, where $\hat{x} \geq 0$ is an estimate of the solution, minimizing the 2-norm $\|Wx\|_2$ is equivalent to minimizing the cumulative dose over all voxels, $\|Ax\|_1$, because

$$\| Wx \|_2^2 = \| \text{diag}(\hat{x})^{-1/2}\text{diag}(A^Te)^{1/2}x \|_2^2$$

$$= \sum_i ((\sum_j A_{ij})^{1/2}x_i\hat{x}_i^{-1/2})^2 \neg \sum_i (\sum_j A_{ij})x_i = \| Ax \|_1$$

with equality at $\hat{x} = x$.

Some embodiments of the invention are based on another realization that the dual of the LDP problem is a a non-negative quadratic program (NNQP) of a form that can be efficiently solved via parallel quadratic programming (PQP) iterations, especially on parallel hardware such as a graphic processing unit (GPU). The PQP is completely parallelizable for any problem data structure, and can readily exploit the full parallelism of multiprocessor machines, including multi-core, single-instruction/multiple data (SIMD) and GPU. The PQP implementation can be reduced to matrix-vector products and a scalar divide, and such simplicity provides considerable speed advantages even when implemented on serial computers.

Accordingly, some embodiments are taking a dual 420 of the LDP transforming the LDP into a non-negative quadratic program (NNQP), and solving 430 the NNQP using a PQP rescaling iteratively a candidate solution of the NNQP to determine a dual solution 435.

Such reformulation of the LDP allows determining the optimal and feasible solution in real time, which provides clinicians with unique opportunity to explore effects of different constraints on diagnostic parameters in real time. For example, in one embodiment, the PQP iterations are specialized to the LDP dual by splitting the dual variables into two or more groups, typically one group representing upper-bound constraints and one group representing lower-bound constraints to further increase the computational efficiency.

Geometrically, this is an operation in the very high-dimensional space of dual variables (one variable for each constraint on the voxel); the constraints are represented by a quadratic cost in this space and the goal is to find a point in the non-negative part of this space that minimizes the quadratic cost. After the dual solution 435 is determined, this dual-optimal point is related to the optimal beam intensity vector via a linear transform. Thus, the method determine 440 a primal solution 445 of the LDP using the dual solution 435 of the NNQP.

Accordingly, some embodiments of the invention solve the optimization problem (2.1) using multiplicative updates of the PQP by converting the LDP problem (2.1) into a non-negative least-squares problem by taking its dual according to

$$\mathbf{y}^* = \min_{\mathbf{y} \geq 0} \; D(\mathbf{y}) \doteq \min_{\mathbf{y} \geq 0} \; \frac{1}{2} \mathbf{y}^T \mathbf{Q} \mathbf{y} - \mathbf{h}^T \mathbf{y}, \tag{2.2}$$

where

$$\mathbf{Q} = \begin{bmatrix} \mathbf{A} \\ -\mathbf{A} \\ \mathbf{I} \end{bmatrix} \mathbf{W}^{-2} \begin{bmatrix} \mathbf{A} \\ -\mathbf{A} \\ \mathbf{I} \end{bmatrix}^T, \tag{2.3}$$

$$\mathbf{h} = \begin{bmatrix} \mathbf{b}_{\min} \\ -\mathbf{b}_{\max} \\ 0 \end{bmatrix}, \tag{2.4}$$

and

$$\mathbf{y} = \begin{bmatrix} \mathbf{u} \\ \mathbf{v} \\ \mathbf{w} \end{bmatrix}. \tag{2.5}$$

The vectors u, v, and w include the dual variables corresponding to the lowerbound, upperbound, and the non-negativity constraints respectively. The PQP algorithm splits matrix Q into non-negative matrices Q → Q+ - Q- and similarly splits vector h, then solves for y* by iterating the linearly convergent fixpoint

$$\mathbf{y} \leftarrow \mathbf{y} \; diag \left( \mathbf{Q}^- \mathbf{y} + \mathbf{h}^+ \right) \; diag \left( \mathbf{Q}^+ \mathbf{y} + \mathbf{h}^- \right)^{-1},$$

where ∘ is the Hadamard (elementwise) product and the inverse operator applies elementwise to vectors. Substituting in the LDP dual variables yields LDP-specific iterations

$$\mathbf{u} \leftarrow \mathbf{u} \circ \left( \mathbf{A} \mathbf{W}^{-2} \mathbf{A}^T \mathbf{v} + \mathbf{b}_{\min} \right) \circ \left( \mathbf{A} \mathbf{W}^{-2} (\mathbf{A}^T \mathbf{u} + \mathbf{w}) \right)^{-1}, \tag{2.6}$$

$$\mathbf{v} \leftarrow \mathbf{v} \circ \left( \mathbf{A} \mathbf{W}^{-2} (\mathbf{A}^T \mathbf{u} + \mathbf{w}) \right) \circ \left( \mathbf{A} \mathbf{W}^{-2} \mathbf{A}^T \mathbf{v} + \mathbf{b}_{\max} \right)^{-1}, \tag{2.7}$$

$$\mathbf{w} \leftarrow \max\left(0, \mathbf{A}^T(\mathbf{v} - \mathbf{u})\right), \qquad (2.8)$$

After the above iterations have converged, the method determines the primal solution by using the formula x* = W$^{-2}$max (0,A$^T$(u* - v*)). The iterations can be further simplified by eliminating w algebraically.

**Balancing two criteria in a slacked formulation**

It is often the case that the prescription given by a clinician is infeasible, which means that the polytope 210 in the LDP primal space does not enclose a positive volume. One solution to this problem is to relax a subset of constraints by allowing some "slack" in their satisfaction. The amount of slack can be penalized in the objective to prevent excessively relaxing constraints. Geometrically, this is equivalent to growing the volume of the polytope by moving some of its defining half-spaces outward. As the polytope changes shape, the position optimal point can be updated as shown in Figure 3.

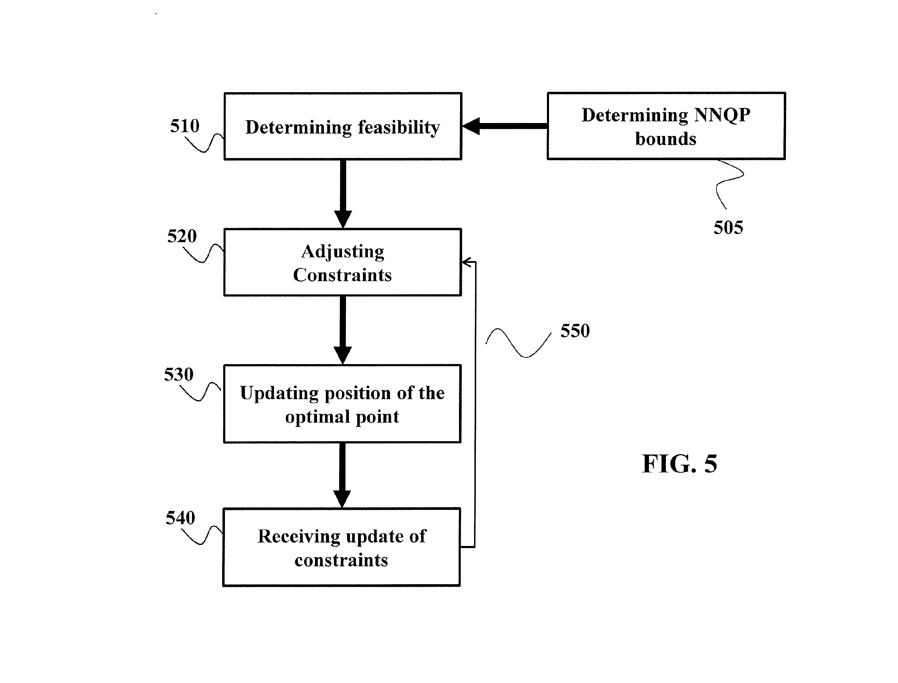

Figure 5 shows a block diagram of a method for updating the position of the point in response to a change of at least one constraint according to some embodiments of the invention. The method determines 510, in response to receiving the constraints on diagnostic parameters, if the polytope is feasible. The method updates 520 at least one constraint automatically, if the polytope is infeasible until the polytope corresponding to the updated constraints is feasible. The optimal point is determined 530 for the feasible polytope.

In addition, some embodiments render the distribution of the dose of radiation with respect to the treatment volume on an output device and provide an interface on the output device for updating 540 at least one constraint. The update causes, in real time, a repeating 550 of the determining the position of the point and the distribution of the dose, and can update the rendering of the distribution of the dose on the output device.

For example, some embodiments are based on another realization that the specialized PQP/LDP iterations can be modified to calculate a set of beam weights that optimally balances between any two quadratic objectives, in particularly, minimizing a measure of total irradiation and a measure of penalized slack.

To that end, one embodiment adds a slack variable s to the upper bounds of some set $v$ of constraints and penalize this slack variable in the objective, yielding optimization problem:

$$\min_{(x,s)\geq 0} \frac{1}{2}\left[(1-\beta)\parallel \mathbf{Wx} \parallel_2^2 + \beta s^2\right] \quad \text{s.t.} \quad \mathbf{b}_{\min} \leq \mathbf{Ax} \leq \mathbf{b}_{\max} + s\mathbf{e}_v, \quad (3.1)$$

where e$_v$ is a vector whose $i$th component is 1 if $i \in v$ and 0 otherwise and $\beta$ is the relative weight on the slack penalty. The objective provide a trade-off between the overall quality of the solution (as measured by radiation cost$\parallel \mathbf{Wx}\parallel$) and minimizing excess dose in the relaxed constraints (as measured by the slack cost $s^2$). The trade-off is controlled by the weight $0 < \beta < 1$.

The dual of the above slacked problem is:

$$\min_{\mathbf{y}\geq 0} \frac{1}{2}\mathbf{y}^T\mathbf{Hy} - \mathbf{h}^T\mathbf{y}, \qquad (3.2)$$

where $\mathbf{H} = \dfrac{1}{1-\beta}\mathbf{Q} + \dfrac{1}{\beta}\mathbf{E}$; Q, h, and y are as defined for the original LDP problem, and

$$E = \begin{bmatrix} 0 \\ \mathbf{e}_{\mathcal{V}} \\ 0 \end{bmatrix} \begin{bmatrix} 0 \\ \mathbf{e}_{\mathcal{V}} \\ 0 \end{bmatrix}^T . \qquad (3.3)$$

In one embodiment, the slack variable s and its associated dual variable t are algebraically eliminated, e.g., by solving the KKT optimal conditions to find that $S = \frac{1}{\beta} v^T e_{\mathcal{V}}$ and t = 0. As with the original LDP problem, the embodiments algebraically determine the matrix split = H$^+$ - H- . The dual problem and the matrix split is used to obtain a set of multiplicative updates for the slacked problem:

$$\mathbf{u} = \mathbf{u} \circ \left( A \frac{W^{-2}}{1-\beta} A^T \mathbf{v} + \mathbf{b}_{\min} \right) \circ \left( A \frac{W^{-2}}{1-\beta} (A^T \mathbf{u} + \mathbf{w}) \right), \qquad (3.4)$$

$$\mathbf{v} = \mathbf{v} \circ \left( A \frac{W^{-2}}{1-\beta} (A^T \mathbf{u} + \mathbf{w}) \right) \circ \left( A \frac{W^{-2}}{1-\beta} A^T \mathbf{v} + s\mathbf{e}_{\mathcal{V}} + \mathbf{b}_{\max} \right), \qquad (3.5)$$

$$\mathbf{w} = \max\left(0, A^T(\mathbf{v} - \mathbf{u})\right), \qquad (3.6)$$

$$s = \frac{1}{\beta} \mathbf{v}^T \mathbf{e}_{\mathcal{V}}. \qquad (3.7)$$

After the above iterations have converged, the embodiment obtain the desired solution through

$$x^* = \frac{W^{-2}}{1-\beta} \max\left(0, A^T(u^* - v^*)\right).$$

A variation on the slacked problem is to let the slack variable s represent the actual value of the relaxed maximum safe dose constraint. In this case, the problem takes the following form:

$$\min_{(\mathbf{x},s) \geq 0} \frac{1}{2}\left[(1-\beta) \| W\mathbf{x} \|_2^2 + \beta s^2\right] \quad \text{s.t.} \quad \mathbf{b}_{\min} \leq A\mathbf{x} \leq \mathbf{b}_{\max} \circ \mathbf{e}_{\mathcal{V}} + s\mathbf{e}_{\mathcal{V}}. (3.8)$$

The derivation of the PQP multiplicative iteration is similar to the original slacked problem, with the difference that $b_{\max}$ is replaced with $b_{\max} \circ e_{\mathcal{V}}$.

Another variation is to include a penalized slack variable on a subset of the lowerbound constraints instead of the upperbounds. The analysis is similar, with the only change being in the matrix E:

$$\mathbf{E} = \begin{bmatrix} \mathbf{e}_{\mathcal{V}} \\ 0 \\ 0 \end{bmatrix} \begin{bmatrix} \mathbf{e}_{\mathcal{V}} \\ 0 \\ 0 \end{bmatrix}^T . \qquad (3.9)$$

Some embodiments also include the PQP iterations for this problem adding a slack on a subset $v$ of the lowerbound constraints:

$$\mathbf{u} = \mathbf{u} \circ \left( \mathbf{A} \frac{\mathbf{W}^{-2}}{1-\beta} \mathbf{A}^T \mathbf{v} + \mathbf{b}_{\min} \right) \circ \left( \mathbf{A} \frac{\mathbf{W}^{-2}}{1-\beta} (\mathbf{A}^T \mathbf{u} + \mathbf{w}) + s\mathbf{e}_{\mathcal{V}} \right), \qquad (3.10)$$

$$\mathbf{v} = \mathbf{v} \circ \left( \mathbf{A} \frac{\mathbf{W}^{-2}}{1-\beta} (\mathbf{A}^T \mathbf{u} + \mathbf{w}) \right) \circ \left( \mathbf{A} \frac{\mathbf{W}^{-2}}{1-\beta} \mathbf{A}^T \mathbf{v} + \mathbf{b}_{\max} \right), \qquad (3.11)$$

$$\mathbf{w} = \max\left( 0, \mathbf{A}^T (\mathbf{v} - \mathbf{u}) \right), \qquad (3.12)$$

$$s = \frac{1}{\beta} \mathbf{u}^T \mathbf{e}_{\mathcal{V}}. \qquad (3.13)$$

and determine **x\*** as described above.

In addition, some embodiments are based on a realization that the primal LDP problem is infeasible if and only if its dual is unbounded, as described in more details below. Accordingly some embodiments determine 510 the feasibility of the LDP by determining 505 bounds for the NNQP and determining that the LDP is infeasible if the NNQP is unbounded.

**Exploring Pareto curve**

During the treatment planning, a set of constraints to relax is selected to examine the set of solutions that become available as larger and larger violations of those constraints are tolerated. Different amounts of slack penalty determine different optimal solutions. For example, the penalty determines a trade-off between minimizing total irradiation and minimizing the amount of slack allowed in certain constraints. The set of all optimal solutions as one varies this trade-off is called a Pareto curve.

The Pareto curve is parameterized by the criterion weighting parameter β. However, β is not a clinically meaningful parameter. Instead, the treatment planner is interested in seeing solutions as a function of the value of the slack variable s, i.e. how much the constraints are relaxed to obtain a optimal solution for a given criterion weighting β. Thus, s can be the outcome of an optimization for a given β. Some embodiments of the invention invert that relationship, and compute the weighting β and solution x\* directly from a constraint relaxation value s.

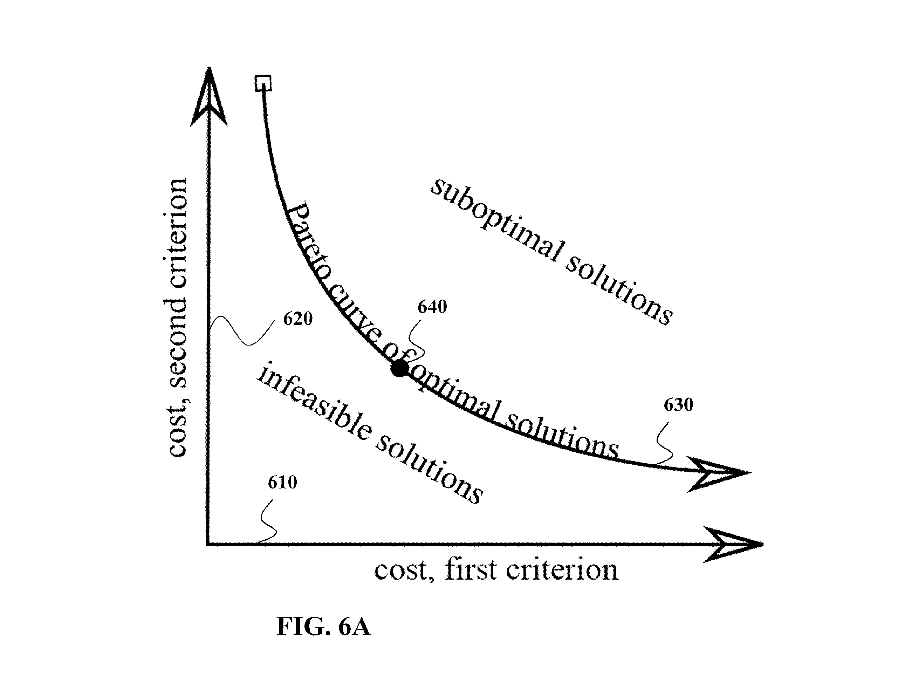

Figure 6A shows an example of a Pareto curve 630. Geometrically, this curve is in a 2D space where axes 610 and 620 represent various costs criteria. Every point in this 2D space represents a solution to a slightly different problem. The Pareto curve represents the "best" solutions in the sense that all solutions on one side are inferior and all solutions on the other side are infeasible.

Some embodiments of the invention are based on yet another realization that it is possible to generate any and every solution on this curve, indexed by the slack variable, from a small number of PQP/LDP optimizations. Accordingly, some embodiments determine a Pareto curve representing optimal solutions for various values of the slack variable using interpolation between a set of optimal values determined for a set of slack variables and determine the optimal solution for a specific slack variable using the Pareto surface.

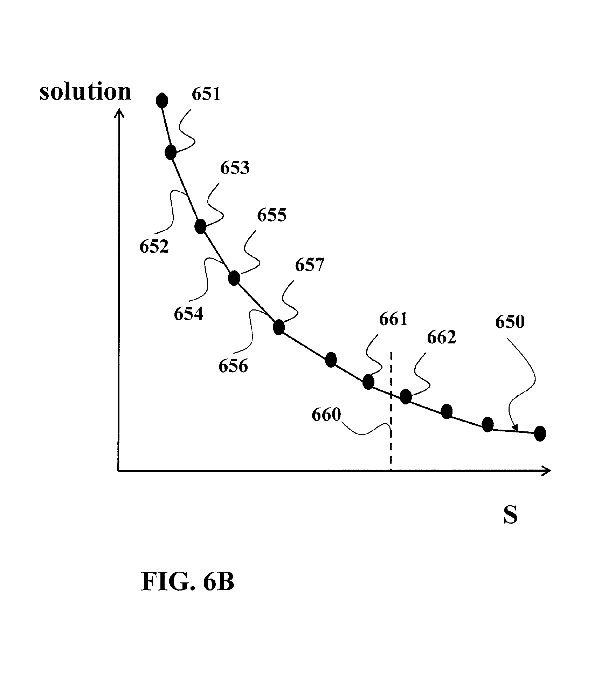

Figure 6B shows an example of such a Pareto curve 650 balancing the values of the slack variable and one of the possible solutions of the optimization, e.g., a value of the diagnostic parameter. The points on the curve 650, e.g., the points 651, 653, 655, 657 are determined by the PQP/LDP iterations. The segments connecting the points, e.g., the segments 652, 654, 656, are determined using interpolation, e.g., by connecting the determined points.

In some embodiments, the solutions and the interpolation of the solution are determined only if needed. For example, if the clinician wants to see an optimal solution 660 that has X units of slack in a particular set of constraints, some embodiments retrieve two optimal solutions 661 and 662 that use more and less than X units of slack, respectively, and that have the same pattern of zeros in their dual solution vector. Then the new optimal solution is a linear interpolation between their primal solution vectors (beam intensities), with the weighting determined from X as shown below.

Because the PQP/LDP iterations are quite fast, these retrieved solutions can be computed on-the-fly if they are not already stored in a memory. Clinically, this means that the clinicians can rapidly explore any Pareto curve associated with any subset of constraints, by requesting optimal solutions corresponding to any amount of slack. Most of the time, the embodiments can provide the optimal solution instantly via piecewise linear interpolation; otherwise, an efficient PQP/LDP iteration can be employed to add a solution to the cache that extends the range of interpolatable solutions.

According to the structure of the Pareto curve, as the criterion weighting parameter $\beta$ changes, the dose distribution changes, and eventually the set of voxels of the treatment volume that receive their maximum or minimum doses changes. This reflects a change in the subset of constraints that are active in the solution.

Consequently the range $\beta \in (0,1)$ can be partitioned into intervals in which the membership of the active set remains constant even though the solution changes with $\beta$. If the active constraints for a particular value of $\beta$ are known, the slacked optimization problem (3.1) can be rewritten using only the constraints that are active

$$\min_{(x,s)\geq 0} \frac{1}{2}\left\|\begin{bmatrix}\sqrt{1-\beta}\,\mathbf{W} & 0\\ 0 & \sqrt{\beta}\end{bmatrix}\begin{bmatrix}\mathbf{x}\\ s\end{bmatrix}\right\|_2^2 \quad \text{s.t.} \quad [\mathbf{B}_1 \quad \mathbf{B}_2]\begin{bmatrix}\mathbf{x}\\ s\end{bmatrix} = \mathbf{c}, \qquad (4.1)$$

where the matrix $\mathbf{B_1}$, the matrix $\mathbf{B_2}$, and the vector c form the set of active constraints (indicated by positive-valued variables in the dual optimum solution).

The [$B_1$ $B_2$] have full row rank, otherwise, the active constraints are redundant. Because the problem in equation (4.1) is a non-negative weighted least squares problem, some embodiments can write the closed form solution as:

$$\begin{bmatrix}\mathbf{x}\\ s\end{bmatrix} = \begin{bmatrix}\dfrac{1}{1-\beta}\mathbf{W}^{-2} & 0\\ 0 & \dfrac{1}{\beta}\end{bmatrix}\begin{bmatrix}\mathbf{B}_1^T\\ \mathbf{B}_2^T\end{bmatrix}\left([\mathbf{B}_1 \quad \mathbf{B}_2]\begin{bmatrix}\dfrac{1}{1-\beta}\mathbf{W}^{-2} & 0\\ 0 & \dfrac{1}{\beta}\end{bmatrix}\begin{bmatrix}\mathbf{B}_1^T\\ \mathbf{B}_2^T\end{bmatrix}\right)^{-1}\mathbf{c}$$

$$= \begin{bmatrix}\dfrac{1}{1-\beta}\mathbf{W}^{-2}\mathbf{B}_1^T\\ \dfrac{1}{\beta}\mathbf{B}_2^T\end{bmatrix}\left(\dfrac{1}{1-\beta}\mathbf{B}_1\mathbf{W}^{-2}\mathbf{B}_1^T + \dfrac{1}{\beta}\mathbf{B}_2\mathbf{B}_2^T\right)^{-1}\mathbf{c}.$$

Observing that $\mathbf{B}_2\mathbf{B}_2^T$ is a rank 1 matrix, one embodiment use the matrix inversion lemma to obtain the following equations for x and s:

$$\mathbf{x} = \mathbf{W}^{-2}\mathbf{B}_1^T(\mathbf{B}_1\mathbf{W}^{-2}\mathbf{B}_1^T)^{-1}(\mathbf{c} - s\mathbf{B}_2) \qquad (4.2)$$

and

$$s = \frac{aq}{1-bq}, \qquad (4.3)$$

with ratio

$$q = \frac{1-\beta}{\beta}; \quad \beta = \frac{1}{1+q}, \qquad (4.4)$$

and constants

$$a = \mathbf{B}_2^T(\mathbf{B}_1\mathbf{W}^{-2}\mathbf{B}_1^T)^{-1}\mathbf{c}, \qquad (4.5)$$

and

$$b = -\mathbf{B}_2^T(\mathbf{B}_1\mathbf{W}^{-2}\mathbf{B}_1^T)^{-1}\mathbf{B}_2. \qquad (4.6)$$

The matrix inversion in the equations for x, a, b is large and rank-deficient, but is not computed. The slack s depends linearly on known q and two unknown constants, a and b. Thus, for any interval $\beta \in (\beta_1, \beta_2)$ in which the set of active constraints in the optimal LDP solution is unchanged, two solutions suffice to determine a, b and thus all solutions in the $\beta$ interval where the active set is unchanged.

According to equation (4.3) and two solutions $(\beta_1, s_1)$ and $(\beta_2, s_2)$:

$$\begin{bmatrix} a \\ b \end{bmatrix} = \begin{bmatrix} q_1 & q_1 s_1 \\ q_2 & q_2 s_2 \end{bmatrix}^{-1} \begin{bmatrix} s_1 \\ s_2 \end{bmatrix}, \qquad (4.7)$$

where $q_1$ and $q_2$ are obtained from $\beta_1$ and $\beta_2$ respectively via equation (4.4).

After the parameters a and b are known for a specific active set:

$$s = \frac{a(1-\beta)}{\beta - b(1-\beta)}, \qquad (4.8)$$

and

$$\beta = \frac{a+bs}{a+bs+s}. \qquad (4.9)$$

To obtain the solution x corresponding to a given weighting $\beta$ or slack s, the equation (4.2) can be written as

$$\mathbf{x} = \mathbf{g} + s\mathbf{h}, \qquad (4.10)$$

where g derives from the unslacked problem and h is a slack-induced correction:

$$\mathbf{g} = \mathbf{W}^{-2}\mathbf{B}_1^T(\mathbf{B}_1\mathbf{W}^{-2}\mathbf{B}_1^T)^{-1}\mathbf{c},$$

$$\mathbf{h} = -\mathbf{W}^{-2}\mathbf{B}_1^T(\mathbf{B}_1\mathbf{W}^{-2}\mathbf{B}_1^T)^{-1}\mathbf{B}_2.$$

These vectors can be computed without any matrix operations. Given two solutions $x_1$ and $x_2$ with the same active set but objective weightings $\beta_1$ and $\beta_2$, manipulation of equation (4.2) yields

$$\mathbf{h} = (\mathbf{x}_1 - \mathbf{x}_2)/(s_1 - s_2), \text{ and} \qquad (4.11)$$

$$\mathbf{g} = \mathbf{x}_1 - s_1\mathbf{h}. \qquad (4.12)$$

Due to the convexity of the original problem, the equation (4.10) parameterizes a facet of the Pareto slice and the endpoints of this facet can be found by solving for minimal and maximal values of s where x = g + sh and Ax = Ag + sAh satisfy the problem constraints, which is an easy calculation for PBDO as most of these constraints are box constraints. Each endpoint is shared by an adjoining facet, so performing one optimization slightly beyond the endpoint gives enough information to characterize this next facet. Therefore the entire Pareto slice can be characterized at a cost of one optimizations per facet. Then as the treatment planner requests solutions corresponding to different relaxation values s, these can be generated on-the-fly by identifying the appropriate facet and applying equation (4.10).

**Acceleration techniques**

Some embodiments reduce the PQP iterations by periodically or intermittently applying an acceleration technique in a specific subspace of the optimization problem space. Those embodiments are based on a recognition that a various factors can slow down the convergence of PQP. For example, when some elements of the vector representing the current candidate solution of the PQP are close to zero, or scaling factors of the PQP iteration are close to one, the change of the candidate solution can be very small.

To avoid such problem, some embodiments update the value of the candidate solution by a different type of operation, i.e., not scaling. Acceleration techniques of one embodiment update the candidate solution by finding an update direction and a step length along that direction. The typical technique uses a negative cost function gradient direction, and performs an optimal step selection. For example, some embodiments determine gradient of the convex quadratic function to be minimized by dual problem at the candidate solution.

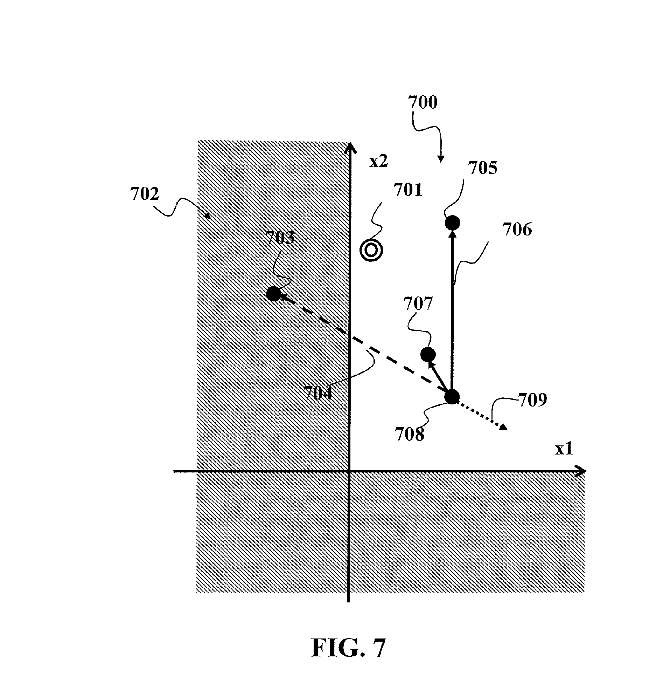

Figure 7 shows a graph comparing a difference between PQP update iteration, and various acceleration techniques according to some embodiments of the invention. The update 707 of a candidate solution 708 can be obtained by the iteration of the PQP towards the optimal solution 701. The update 707 remains in the feasible region 700 of the solutions but may be very short, i.e., the difference between the solutions 708 and 707 is small.

One embodiment considered determines the updated solution 703 with acceleration techniques using a decreasing direction 704, which is the negative of the cost function gradient 709. However, the change of the candidate value according to the gradient may take the candidate solution outside of feasible region of the dual problem. And, if the solution goes into the unfeasible region, the PQP method breaks. For example, the solution may leave the feasible region 700 and enter the unfeasible region 702.

However, some embodiment are based on another realization that in contrast with the feasible region of the primal problem, which depends on the clinical constraints, the feasible region of the dual problem is always the much simpler non-negative cone, which is the set of vectors that have no negative elements. Thus, if the anti-gradient of the NNQP is represented by its components, some components may point toward the unfeasible region, and some components may point toward the feasible region, i.e., the direction where the positive cone is unbounded. Thus, some embodiments select only the components of the anti-gradient that points toward the feasible region to update the candidate solution. Such modification of the anti-gradient ensures that the candidate solution always stays in the feasible region.

For example, one embodiment occasionally solve, in closed form, for the lowest-cost point along the half-line that runs from positive infinity to the current NNQP solution estimate along any direction that is a strictly nonpositive subgradient. This point is known to be interior to the feasible region, therefore it moves near-zero variables away from zero. Any strictly nonpositive subgradient at the current estimate x offers a 1D affine subspace with this property, because by construction any descent along the subgradient moves further into the nonnegative cone, which is the dual feasible region.

One embodiment selects the subgradient as the component of the gradient that is negative. For the original LDP problem (2.1), let the vector g to be the negated gradient of the dual objective:

$$\mathbf{g} = \mathbf{h} - \mathbf{Q}\mathbf{y} = \begin{bmatrix} \mathbf{g}_u \\ \mathbf{g}_v \\ \mathbf{g}_w \end{bmatrix} = \begin{bmatrix} \mathbf{b}_{\min} - \mathbf{A}\mathbf{x} \\ \mathbf{A}\mathbf{x} - \mathbf{b}_{\max} \\ -\mathbf{x} \end{bmatrix}, \qquad (6.1)$$

which happens to measure primal constraint violations wherever **g** > **0**. Thus the subgradient

$$\mathbf{d} = \max(\mathbf{g}, 0) = \begin{bmatrix} \mathbf{d}_u \\ \mathbf{d}_v \\ \mathbf{d}_w \end{bmatrix} = \begin{bmatrix} \max(\mathbf{b}_{\min} - \mathbf{A}\mathbf{x}, 0) \\ \max(\mathbf{A}\mathbf{x} - \mathbf{b}_{\max}, 0) \\ \max(-\mathbf{x}, 0) \end{bmatrix} \qquad (6.2)$$

points in the direction where dual variables need to be grown away from zero to bolster unsatisfied constraints. Since the objective (2.1) is quadratic, the optimum along **d** can be solved for algebraically; it is

$$\mathbf{y} + \alpha\mathbf{d} \quad with \quad \alpha = \frac{\mathbf{g}^T\mathbf{d}}{\mathbf{d}^T\mathbf{Q}\mathbf{d}} = \frac{\mathbf{g}_u^T\mathbf{d}_u + \mathbf{g}_v^T\mathbf{d}_v}{\mathbf{r}^T\mathbf{r}}, \qquad (6.3)$$

and

$$\mathbf{r} = \mathbf{W}^{-1} \begin{bmatrix} \mathbf{A} \\ -\mathbf{A} \\ \mathbf{I} \end{bmatrix}^T \mathbf{d} = \mathbf{W}^{-1}(\mathbf{A}^T(\mathbf{d}_u - \mathbf{d}_v) + \mathbf{d}_w). \qquad (6.4)$$

The convergence of the mixed PQP iterations follows from the PQP convergence analysis. Assume PQP updates are used infinitely often. Then, the PQP-S updates converge monotonically to the minimum of the dual problem. Below, is an example of a stepsize $\alpha$ used in the PQP-S algorithm.

For the upperbound slack problem (3.1), the results can be derived as

$$\mathbf{g} = \mathbf{h} - \mathbf{Hy} = \begin{bmatrix} \mathbf{g}_u \\ \mathbf{g}_v \\ \mathbf{g}_w \end{bmatrix} = \begin{bmatrix} \mathbf{b}_{\min} - \mathbf{Ax} \\ \mathbf{Ax} - \mathbf{b}_{\max} - s\mathbf{e}_{\mathcal{V}} \\ -\mathbf{x} \end{bmatrix}. \tag{6.5}$$

$$\alpha = \frac{\mathbf{g}^T \mathbf{d}}{\mathbf{d}^T \mathbf{Hd}} = \frac{\mathbf{g}_u^T \mathbf{d}_u + \mathbf{g}_v^T \mathbf{d}_v}{\frac{1}{1-\beta} \mathbf{r}^T \mathbf{r} + \frac{1}{\beta}(\mathbf{d}_v^T \mathbf{e}_{\mathcal{V}})^2}, \tag{6.6}$$

where $\mathbf{d} = \max(\mathbf{g}, 0)$ and $\mathbf{r}$ is previously defined. Other variations of the slack problem can be similarly accelerated.

**Infeasibility detection**

If the LDP problem is primal feasible, the PQP converges to an optimal point. However, there is a need for a method for determining infeasible problems, which are very common in radiation therapy planning. Some embodiments are based on a realization that the primal LDP problem is infeasible if and only if (iff) its dual is unbounded.

*Proof.* The primal LDP problem is infeasible iff the following linear program (LP) is infeasible:

$$\begin{array}{ll} \underset{x}{\text{minimize}} & 0 \\ \text{subject to} & \begin{bmatrix} \mathbf{A} \\ -\mathbf{A} \\ \mathbf{I} \end{bmatrix} \mathbf{x} \geq \begin{bmatrix} \mathbf{b} \\ -\mathbf{c} \\ 0. \end{bmatrix} \end{array} \tag{7.1}$$

The dual of the above LP is:

$$\begin{array}{ll} \underset{\lambda}{\text{maximize}} & \lambda^T \begin{bmatrix} \mathbf{b} \\ -\mathbf{c} \\ 0 \end{bmatrix} \\ \text{subject to} & \lambda^T \begin{bmatrix} \mathbf{A} \\ -\mathbf{A} \\ \mathbf{I} \end{bmatrix} = 0 \\ & \lambda \geq 0. \end{array} \tag{7.2}$$

This dual form is always feasible. According to Farkas' Lemma, the primal LDP problem is infeasible iff $\exists \lambda \geq 0$ such that the following equations are satisfied:

$$\lambda^T \begin{bmatrix} \mathbf{A} \\ -\mathbf{A} \\ \mathbf{I} \end{bmatrix} = \mathbf{0}, \qquad \lambda^T \begin{bmatrix} \mathbf{b} \\ -\mathbf{c} \\ 0 \end{bmatrix} > 0. \quad (7.3)$$

or equivalently

$$\mathbf{A}^T (\lambda_u - \lambda_v) \le \mathbf{0}, \qquad \mathbf{b}^T \lambda_u > \mathbf{c}^T \lambda_v. \tag{7.4}$$

If the PQP computes such a $\lambda$, the method can terminate with a certificate of infeasibility. Thus, the dual form (7.2) is unbounded iff primal LP (7.1) is infeasible. If there is no $\lambda \ge 0$ that satisfies (7.3), then clearly the optimum of the dual LP problem, if it exists, is upper bounded by 0 and therefore not unbounded. For the other direction, if $\exists \lambda \ge 0$ satisfying (7.8), the embodiments can select an increasingly large constant a so that a$\lambda$ result in the dual LP objective to be arbitrarily large (resulting in an unbounded objective). Finally, the last step is to show that the dual of the LDP problem is unbounded iff $\exists \lambda \ge 0$ that satisfies (7.8).

The backward direction ($\Leftarrow$) is proved with the same argument used for proving the unboundedness of the LP dual. For the forward direction, we suppose the LDP dual is unbounded. Then, there must exist some $\lambda \ge 0$ in the null space of Q that satisfies $h^T \lambda > 0$. This condition on $\lambda$ is equivalent to the one given in (7.8).

The above proposition guarantees that as long as an upper bound on the dual objective is satisfied by all primal feasible problems, the PQP algorithm is able to detect infeasibility. A better upperbound however results in faster detection. Thus, one embodiment assume that an upperbound U for Ax can be obtained. For radiation therapy, an example of such an upperbound could be to set $U = b_{max}$, place a limit on the maximum possible dose to tumor voxels, and similarly place another limit on the maximum possible dose to normal tissue and OARs.

If $\mathbf{W} = \mathrm{diag}(\mathbf{A}^T \mathbf{e})$, then

$$\| \mathbf{Wx} \|_1 = \sum_j \left( \sum_i a_{ij} \right) x_j \tag{7.5}$$

$$= \sum_i \sum_j a_{ij} x_j \tag{7.6}$$

$$= \| \mathbf{Ax} \|_1. \tag{7.7}$$

Next, the embodiment compute an upperbound on the dual objective using the following sequence of inequalities:

$$\text{dualobjective} \le \frac{1}{2} \| \mathbf{Wx} \|_2^2 \tag{7.8}$$

$$\leq \frac{1}{2\sqrt{n}} \parallel \mathbf{Wx} \parallel_1^2 \qquad\qquad (7.9)$$

$$= \frac{1}{2\sqrt{n}} \parallel \mathbf{Ax} \parallel_1^2 \qquad\qquad (7.10)$$

$$\leq \frac{1}{2\sqrt{n}} \parallel \mathbf{U} \parallel_1^2, \qquad\qquad (7.11)$$

where in (7.8) used duality theory to relate the primal and dual objectives and in (7.9) used a classical norm bound.

**User Interface**

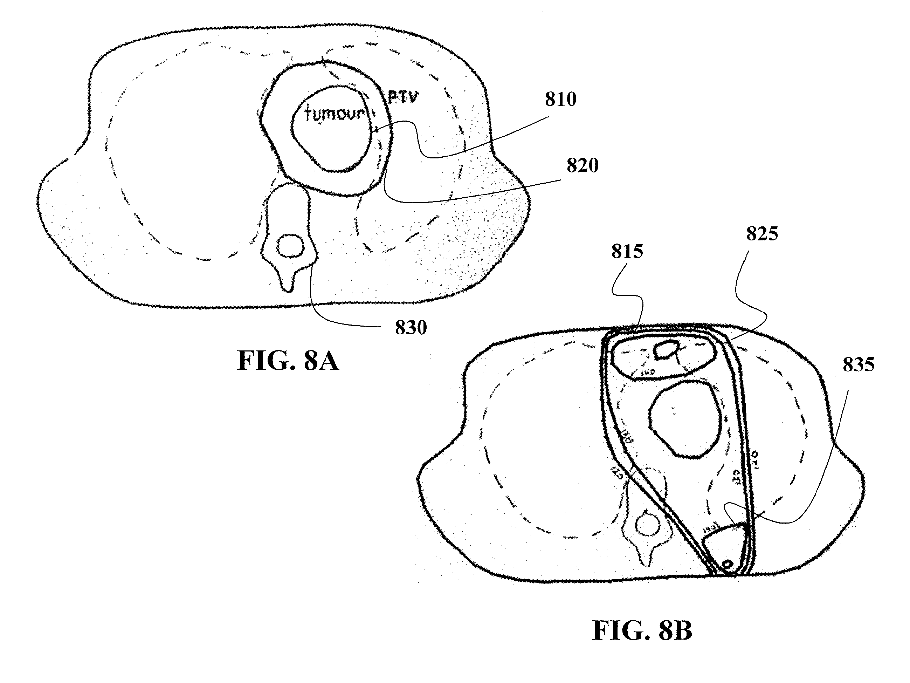

Figures 8A and 8B shows examples of rendering the distribution of the dose of radiation with respect to the treatment volume on an output device. The treatment volume can be rendered to include representation of various organs 830, tissues 820 and/or tumors 810 of a patient. Some embodiments can also render on the output device a representation of the distribution of the dose. For example, the distribution of the dose can be rendered as isocontour 815, 825, 835. In one implementation, the isocontour are colored to indicate different dosage of radiation.

Some embodiments also provide an interface for changing at least one constraint. Such changing causes, in real time, a repeating of the determining the position of the optimal point, the distribution of the dose, and the rendering the distribution of the dose on the output device. Such interface in combination with optimization techniques of various embodiments of the invention allows clinicians to analyze the distribution of the dose of beam of radiation for different constraints on the diagnostic parameters in real time.

The above-described embodiments of the present invention can be implemented in any of numerous ways. For example, the embodiments may be implemented using hardware, software or a combination thereof. When implemented in software, the software code can be executed on any suitable processor or collection of processors, whether provided in a single computer or distributed among multiple computers. Such processors may be implemented as integrated circuits, with one or more processors in an integrated circuit component. Though, a processor may be implemented using circuitry in any suitable format.

Further, it should be appreciated that a computer may be embodied in any of a number of forms, such as a rack-mounted computer, a desktop computer, a laptop computer, minicomputer, or a tablet computer. Also, a computer may have one or more input and output devices. These devices can be used, among other things, to present a user interface. Examples of output devices that can be used to provide a user interface include printers or display screens for visual presentation of output and speakers or other sound generating devices for audible presentation of output.

Examples of input devices that can be used for a user interface include keyboards, and pointing devices, such as mice, touch pads, and digitizing tablets. As another example, a computer may receive input information through speech recognition or in other audible format.

Such computers may be interconnected by one or more networks in any suitable form, including a local area network or a wide area network, such as an enterprise network or the Internet. Such networks may be based on any suitable technology and may operate according to any suitable protocol and may include wireless networks, wired networks or fiber optic networks.

Also, the embodiments of the invention may be embodied as a method, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

**Claims**

1.  A method for optimizing a dose of radiation for a radio-therapy treatment subject to constraints on diagnostic parameters of the radio-therapy treatment, comprising:

    determining a point of a polytope (210) arranged in a coordinate system (220) of the diagnostic parameters, such that a position of the point in the coordinate system (220) is determined at least in part by values of each diagnostic parameter, wherein the polytope (210) is convex with boundaries formed by intersecting half-spaces of feasible values of each diagnostic parameter specified by the constraints, and wherein the point is the closest point (237) of the polytope (210) to an origin (230) of the coordinate system (220) with regard to a weighted Euclidean distance norm; and
    determining a distribution of the dose of radiation for the radio-therapy treatment using the values of the diagnostic parameters corresponding to the position of the point, wherein steps of the method are performed by a processor (401).

2.  The method of claim 1, wherein the constraints on the diagnostic parameters include one or combination of a constraint on a minimal dose of radiation of a tumor (810), a constraint on a maximal dose of radiation of at least one organ-at-risk (OAR), and maximum and minimum constraints on cumulative dose of radiation of tissues (820), and wherein an origin (230) of the coordinate system (220) represents the radio-therapy treatment with a zero dose of radiation.

3.  The method of claim 1, wherein the position of the optimal point is determined in real time in response to receiving the constraints, and without constructing the polytope (210).

4.  The method of claim 1, further comprising:

    updating the position of the point in response to a change of at least one constraint.

5.  The method of claim 1, further comprising:

    determining, in response to receiving the constraints, if the polytope (210) is feasible;
    updating at least one constraint automatically, if the polytope (210) is infeasible; and
    repeating the updating until the polytope (210) corresponding to the updated constraints is feasible.

6.  The method of claim 5, further comprising:

    rendering the distribution of the dose of radiation with respect to the treatment volume on an output device; and
    providing an interface (109) on the output device for changing at least one constraint, such that the changing causes, in real time, a repeating of the determining the position of the optimal point and the distribution of the dose, and the rendering the distribution of the dose on the output device.

7.  The method of claim 1, wherein the position of the point is determined by solving an optimization problem without constructing the polytope (210), comprising:

    formulating a least-distance problem (LDP) minimizing intensity values of beams of radiation subject to minimal and maximal constraints on total dose of radiation of various voxels of a treatment volume;
    taking a dual of the LDP to transform the LDP into a non-negative quadratic program (NNQP);
    solving the NNQP using a parallel quadratic programming (PQP) rescaling iteratively a candidate solution of the NNQP to determine a dual solution (435); and
    determining a primal solution (445) of the LDP using the dual solution (435) of the NNQP.

8.  The method of claim 7, further comprising:

    determining the minimal and maximal constraints on the total dose of radiation of the various voxels of the treatment volume based on the constraints and a voxel map of the treatment volume.

9.  The method of claim 7, further comprising:

adding a slack variable to some constraints for at least some voxels; and
penalizing the slack variable in the formulation of the LDP.

**10.** The method of claim 9, further comprising:

determining a Pareto curve representing optimal solutions for various values of the slack variable using interpolation between a set of optimal values determined for a set of slack variables; and
determining the optimal solution for a specific slack variable using the Pareto curve.

**11.** The method of claim 10, further comprising:

determining, in real-time in response to receiving a value of the slack variable, a part of the Pareto curve including the value of the slack variable.

**12.** The method of claim 7, further comprising:

determining bounds for the objective value of the NNQP; and
determining that the LDP is infeasible if the PQP iterations produce a solution estimate whose objective value exceeds these bounds.

**13.** The method of claim 7, further comprising:

modifying a value of the candidate dual solution of an intermediate iteration with an operation different from the rescaling.

**14.** The method of claim 13, further comprising:

modifying the value of the candidate dual solution in a direction indicated by a negative component of the cost function gradient.

**15.** A method for optimizing a dose of radiation for a radio-therapy treatment of a treatment volume of a patient subject to constraints on diagnostic parameters of the treatment volume, comprising:

determining minimal and maximal constraints on total dose of radiation of each voxel in the treatment volume based on the constraints and a voxel map of the treatment volume;
formulating a least-distance problem (LDP) minimizing intensity values of beams of radiation subject to the minimal and the maximal constraints on the total dose of radiation of each voxel in the treatment volume;
taking a dual of the LDP to transform the LDP into a non-negative quadratic program (NNQP);
solving the NNQP using a parallel quadratic programming (PQP) rescaling iteratively a candidate solution of the NNQP to determine a dual solution (435); and
determining a primal solution (445) of the LDP using the dual solution (435) of the NNQP, wherein steps of the method are performed by a processor (401).

**16.** The method of claim 15, further comprising:

adding a slack variable to some constraints for at least some voxels; and
penalizing the slack variable in the formulation of the LDP.

**17.** The method of claim 16, further comprising:

determining a Pareto curve representing optimal solutions for various values of the slack variable using interpolation between a set of optimal values determined for a set of slack variables; and
determining the optimal solution for a specific slack variable using the Pareto curve.

**18.** The method of claim 17, wherein the processor (401) is a parallel processor (102).

**19.** A radiation therapy system (100), comprising a processor (401) for optimizing a dose of radiation for a radio-therapy treatment of a treatment volume of a patient subject to constraints on diagnostic parameters of the treatment volume, wherein the processor (401) is configured for determining a solution of a least-distance problem (LDP) minimizing intensity values of beams of radiation subject to minimal and maximal constraints on the dose of radiation of each voxel in the treatment volume to produce optimal values of the diagnostic parameters satisfying the constraints, and for determining a distribution of the dose of radiation for the radio-therapy treatment using the optimal values of the diagnostic parameters.

20. The system of claim 19, wherein the processor (401) is a parallel processor (102) and solves the LDP by transforming the LDP into a non-negative quadratic program (NNQP) and solving the NNQP using a parallel quadratic programming (PQP) rescaling iteratively a candidate solution of the NNQP using the parallel processor (102).

**FIG. 1**

FIG. 2

FIG. 3

```
            Determining          ←——   Voxels Constraints on
410            LDP                      total irradiation
                                                                    405
              │
              ▼
420        Taking a dual                 Combining diagnostic
           transforming into NNQP        constraints with
                                         segmentation
              │                                                     415
              ▼
430        Determining dual    ———→        435
           solution of NNQP
                                          Dual solution
              │                              │
              ▼                              │
440        Determining primal   ←————————————
           solution of LDP
              │
              ▼
445        Solution for LDP              FIG. 4
```

401

EP 2 899 659 A1

```
                    ┌──────────────────────────┐        ┌──────────────────────────┐
  510 ⟋             │  Determining feasibility  │◄───────│    Determining NNQP       │
                    └──────────────────────────┘        │         bounds            │
                               │                         └──────────────────────────┘
                               │                                       │
                               ▼                                      505
  520 ⟋             ┌──────────────────────────┐
                    │       Adjusting          │◄─┐
                    │      Constraints         │  │
                    └──────────────────────────┘  │
                               │                   │      ⟋ 550
                               ▼                   │
  530 ⟋             ┌──────────────────────────┐  │
                    │  Updating position of the │  │
                    │      optimal point        │  │
                    └──────────────────────────┘  │
                               │                   │
                               ▼                   │
  540 ⟋             ┌──────────────────────────┐  │
                    │   Receiving update of     │──┘
                    │      constraints          │
                    └──────────────────────────┘
```

# FIG. 5

EP 2 899 659 A1

**FIG. 6A**

FIG. 6B

EP 2 899 659 A1

**FIG. 7**

FIG. 8A

FIG. 8B

EP 2 899 659 A1

Europäisches
Patentamt
European
Patent Office
Office européen
des brevets

## EUROPEAN SEARCH REPORT

Application Number

EP 15 15 1834

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2009/037150 A1 (CRAFT DAVID [US] ET AL) 5 February 2009 (2009-02-05) <br> * abstract * <br> * paragraph [0005] - paragraph [0009] * <br> * paragraph [0019] - paragraph [0033] * <br> * paragraph [0055] - paragraph [0059] * <br> * claims 1-4 * <br> * figures 1-4 * | 1-20 | INV. <br> G06F19/00 <br> A61N5/10 |
| X | US 2011/291028 A1 (BRAND MATTHEW [US]) 1 December 2011 (2011-12-01) <br> * abstract * <br> * paragraph [0002] - paragraph [0051] * <br> * paragraph [0069] - paragraph [0073] * <br> * claim 1 * <br> * figure 1 * | 1-20 | |
| X | BORTFELD ET AL: "Optimized planning using physical objectives and constraints", SEMINARS IN RADIATION ONCOLOGY, SAUNDERS, PHILADELPHIA, PA, US, vol. 9, no. 1, 1 January 1999 (1999-01-01) , pages 20-34, XP005454727, ISSN: 1053-4296, DOI: 10.1016/S1053-4296(99)80052-6 <br> * abstract * <br> * page 21, right-hand column, last paragraph - page 28, left-hand column, paragraph 1 * <br> * page 32, left-hand column, paragraph 2 - page 28, right-hand column, last paragraph * <br> * figures 1,2 * | 1-20 | TECHNICAL FIELDS SEARCHED (IPC) <br><br> G06F <br> A61N |

-/--

The present search report has been drawn up for all claims

| Place of search | Date of completion of the search | Examiner |
|---|---|---|
| Munich | 17 June 2015 | Hilbig, Matthias |

EPO FORM 1503 03.82 (P04C01)

Europäisches Patentamt

European Patent Office

Office européen des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Matthias Hilbig ET AL: "Entwicklung eines inversen Bestrahlungsplanungssystems mit linearer Optimierung", Medizinische Physik, 1 January 2002 (2002-01-01), pages 89-96, XP055196046, Retrieved from the Internet: URL:http://www.sciencedirect.com/science/article/pii/S0939388915704514/pdf?md5=9ecdb1d69c357d250d8ebe9aa420f504&pid=1-s2.0-S0939388915704514-main.pdf [retrieved on 2015-06-16] * the whole document * | 1-20 | |

TECHNICAL FIELDS SEARCHED (IPC)

The present search report has been drawn up for all claims

| Place of search | Date of completion of the search | Examiner |
|---|---|---|
| Munich | 17 June 2015 | Hilbig, Matthias |

EPO FORM 1503 03.82 (P04C01)

## ANNEX TO THE EUROPEAN SEARCH REPORT
## ON EUROPEAN PATENT APPLICATION NO.

EP 15 15 1834

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2015

| Patent document cited in search report | Publication date | Patent family member(s) | | Publication date |
|---|---|---|---|---|
| US 2009037150 A1 | 05-02-2009 | NONE | | |
| US 2011291028 A1 | 01-12-2011 | CN | 102711913 A | 03-10-2012 |
| | | EP | 2456521 A1 | 30-05-2012 |
| | | JP | 5063828 B1 | 31-10-2012 |
| | | JP | 2013500048 A | 07-01-2013 |
| | | TW | 201200196 A | 01-01-2012 |
| | | US | 2011291028 A1 | 01-12-2011 |
| | | WO | 2011148800 A1 | 01-12-2011 |

EPO FORM P0459

For more details about this annex : see Official Journal of the European Patent Office, No. 12/82

**EP 2 899 659 A1**

**Patent documents cited in the description**

- US 8315357 B
- US 2009037150 A